# EUROPEAN PATENT SPECIFICATION

(11) **EP 2 214 699 B1**
(45) Date of publication and mention of the grant of the patent: **30.11.2016**
(21) Application number: 08845718.9
(22) Date of filing: 31.10.2008
(51) Int. Cl.: A61K 38/57, C07K 1/16, C07K 14/81

(54) **METHOD, COMPOSITION, AND ARTICLE OF MANUFACTURE FOR PROVIDING ALPHA-1 ANTITRYPSIN**
VERFAHREN, ZUSAMMENSETZUNG UND HERSTELLUNGSARTIKEL FÜR BEREITSTELLUNG VON ALPHA-1-ANTITRYPSIN
PROCÉDÉ, COMPOSITION ET ARTICLE FABRIQUÉ POUR ADMINISTRER L'ALPHA 1-ANTITRYPSINE

(30) Priority: 02.11.2007 US 984975 P
(43) Date of publication of application: 11.08.2010
(73) Proprietor: Grifols Therapeutics Inc., Research Triangle Park, NC 27709 (US)
(72) Inventor: ARORA, Vikram, Morrisville North Carolina 27560 (US); PAMARTHI, Mohan, Cary North Carolina 27519 (US); SCUDERI, Philip, Chapel Hill North Carolina 27516 (US)
(74) Representative: Durán Moya, Luis-Alfonso
(86) International application number: PCT/US2008/081911
(87) International publication number: WO 2009/059082

(56) References cited:
- WO-A1-02/48176
- WO-A1-2005/014648
- WO-A1-2005/047323
- KR-A- 20040 045 528
- PIITULAINEN EEVA ET AL: "Tailored pharmacokinetic dosing allows self-administration and reduces the cost of IV augmentation therapy with human alpha1-antitrypsin" EUROPEAN JOURNAL OF CLINICAL PHARMACOLOGY, SPRINGER VERLAG, DE LNKD- DOI:10.1007/S00228-003-0589-Z, vol. 59, no. 2, 1 June 2003 (2003-06-01), pages 151-156, XP009140757 ISSN: 0031-6970 [retrieved on 2004-02-19]
- STOLLER JAMES K: "Augmentation therapy for severe alpha 1-antitrypsin deficiency: Is the jury still out on a trial?" THORAX, BMJ PUBLISHING GROUP, GB LNKD- DOI:10.1136/THX.53.12.1007, vol. 53, no. 12, 1 December 1998 (1998-12-01), pages 1007-1009, XP009140759 ISSN: 0040-6376
- HOUCK J C ET AL: "Permeability factor contaminating hyaluronidase preparations" INFLAMMATION, PLENUM PRESS, NEW YORK, NY, US LNKD- DOI:10.1007/BF00913502, vol. 3, no. 4, 1 September 1979 (1979-09-01), pages 447-451, XP009109082 ISSN: 0360-3997
- PIITULAINEN E ET AL.: 'Tailored pharmacokinetic dosing allows self-administration and reduces the cost of IV augmentation therapy with human alpha(I)-antitrypsin.' EUR J CLIN PHARMACOL. vol. 59, no. 2, 01 May 2003, pages 151 - 156, XP009140757
- FAVRE D. ET AL.: 'Hyaluronidase enhances recombinant adeno-associated virus (rAAV)-mediated gene transfer in the rat skeletal muscle.' GENE THERAPY vol. 7, no. 16, August 2000, pages 1417 - 1420, XP009140836
- BRACKERTZ D. ET AL.: 'Proteinase inhibitors in rheumatoid arthritis.' ANN RHEUM DIS. vol. 34, no. 3, June 1975, pages 225 - 230, XP008134196
- SETOGUCHI Y ET AL: "EX VIVO AND IN VIVO GENE TRANSFER TO THE SKIN USING REPLICATION- DEFICIENT RECOMBINANT ADENOVIRUS VECTORS", JOURNAL OF INVESTIGATIVE DERMATOLOGY, NATURE PUBLISHING GROUP, GB, vol. 102, no. 4, 1 April 1994 (1994-04-01) , pages 415-421, XP002044071, ISSN: 0022-202X, DOI: 10.1111/1523-1747.EP12372181

## Description

### FIELD OF THE INVENTION

The present invention relates to alpha-1 antitrypsin (α1-AT) for use in a method of treating or preventing a disorder via a subcutaneous route.

### BACKGROUND OF THE INVENTION

α1-AT deficiency is a relatively common genetic disorder that predisposes affected individuals to liver disease and/or pulmonary emphysema. The most common type of α1-AT deficiency termed protease inhibitor type Z (PiZ), is transmitted as an autosomal recessive trait and affects approximately 1 in 1700 live births in most Northern European and North American populations. The PiZ mutation is a single nucleotide substitution that results in a single amino acid substitution (glutamate 342 to lysine).

It is believed that the major physiological function of α1-AT is the inhibition of neutrophil elastase, cathepsin G, and proteinase 3. The α1-AT produced in individuals with PiZ α1-AT deficiency is functionally active, although there may be a decrease in its specific elastase inhibitory capacity. The predominant site of al-AT synthesis is the liver, however, it is also synthesized in extrahepatic cell types including macrophages, intestinal epithelial cells and intestinal Paneth cells.

The half-life in plasma of type M α1-AT (α1-ATM) (PiM is the normal allotype) is approximately five days. The half-life of the PiZ mutant protein (α1-ATZ) is slightly less, but this difference is insufficient to account for the low plasma levels of al-AT in homozygous PiZ individuals.

The pathogenesis of lung injury in α1-AT deficiency is attributable to the marked reduction in available α1-AT activity. α1-AT has been found to constitute greater than 90% of the neutrophil elastase inhibitor activity in pulmonary alveolar lavage fluid. Thus, it appears that the destructive lung disease seen in many individuals with α1-AT deficiency is due to a perturbation in the net balance between elastase and α1-AT within the lungs. The uninhibited activity of neutrophil elastase, cathepsin G, and proteinase 3, in turn, results in slow destruction of the connective tissue integrity of the lungs. This destruction of connective tissue leads to over distension and a reduction in the retractive force of the lungs which results in decreased expiratory airflow. Smoking exacerbates the problem by causing oxidative inactivation of what α1-AT is present.

At present, treatment options for individuals with pathologies associated with α1-AT deficiency are limited. Liver disease associated with α1-AT deficiency is treated by orthotopic liver transplantation. Perlmutter, Ann. Med. 28:385-94, 1996. Somatic gene therapy to replace the defective α1-AT gene has been discussed, but has yet to be successfully used. Patients with emphysema related to α1-AT deficiency have been treated with purified plasma α1-AT administered intravenously or by intratracheal aerosol administration. See, e.g., U.S. Pat. No. 5,093,316. The efficacy of this treatment regime, however, has yet to be established.

α1-AT supplement therapy has been employed in deficient individuals in the form of repeated intravenous administrations on a chronic basis. This mode of administration can be associated with problems including, for example, the requirement for a healthcare worker for administration, poor venous access, immediate hypersensitive reaction, high cost of the procedure, and wide fluctuations in the plasma levels of α1-AT.

WO 2005/014648 teaches intravenous administration of α1-AT.

There remains a need, therefore, for a method for providing α1**-**AT that at least is easy to administer, is suitable for long-term administration, and achieves desirable α1-AT plasma bioavailability levels.

### SUMMARY OF THE INVENTION

The present invention provides:
Embodiments of the invention are:
   1. Alpha-1 antitrypsin (α1-AT) for use in a method of treating or preventing a disorder or disease associated with α1-AT deficiency in a subject by subcutaneous administration, wherein the therapeutically or prophylactically effective amount of the subcutaneously administered α1-AT is at least about 120% of an intravenously administered therapeutically or prophylactically effective amount of α1-AT wherein the therapeutically or prophylactically effective amount of α1-AT is sufficient to maintain in the subject a blood α1-AT trough level of at least about 80 mg/dL, wherein the therapeutically or prophylactically effective amount of α1-AT is about 60 mg to about 300 mg of α1-AT per kg of body weight of the subject.
   2. α1-AT for use according to embodiment 1, wherein the formulation is a lyophilized formulation to be reconstituted prior to administration.
   3. α1-AT for use according to any one of embodiments 1 or 2, wherein the formulation further comprises a hydrolase.
   4. α1-AT for use according to any one of embodiments 1 or 2, wherein the formulation further comprises a hyaluronidase.

In one embodiment, the therapeutically or prophylactically effective amount of α1-AT is sufficient to maintain a blood α1-AT trough level of at least about 80 mg/dL.

The α1-AT is provided for subcutaneous administration. In one embodiment, the formulation is a lyophilized formulation, wherein prior to administration, the lyophilized formulation can be reconstituted with a suitable aqueous solution, for example an aqueous solution comprising water. Lyophilization and reconstitution of lyophilized formulations are generally known in the art.

In some aspects, α1-AT can be administered subcutaneously for a variety of applications including human and veterinary therapies, either alone or in combination with one or more reagents, e.g., a reagent capable of enhancing the pharmacokinetics and/or pharmacodynamics of the subcutaneously administered α1-AT.

In another aspect, the present specification illustrates a pharmaceutical composition comprising α1-AT and a hyaluronidase, wherein the composition is suitable for subcutaneous administration to a subject.

### BRIEF DESCRIPTION OF THE DRAWINGS

Figure 1 is a graph of plasma alpha-1 antitrypsin (α1-AT) concentration as a function of time after intravenous (IV) or subcutaneous (SC-1 and SC-2) administration.
Figure 2 is a graph of plasma alpha-1 antitrypsin (α1-AT) concentration as a function of time after repeated subcutaneous (SC-3, SC-4, and SC-5) administration.

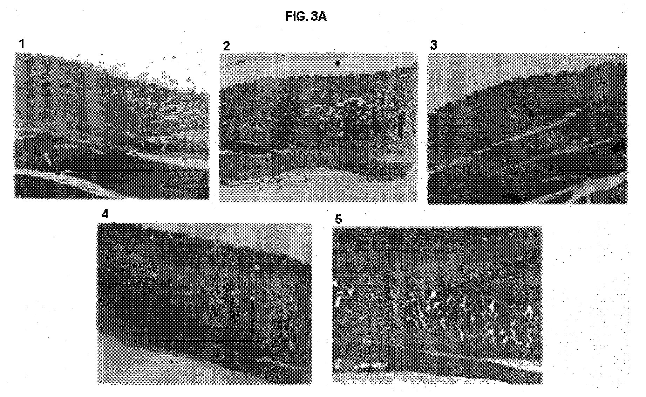

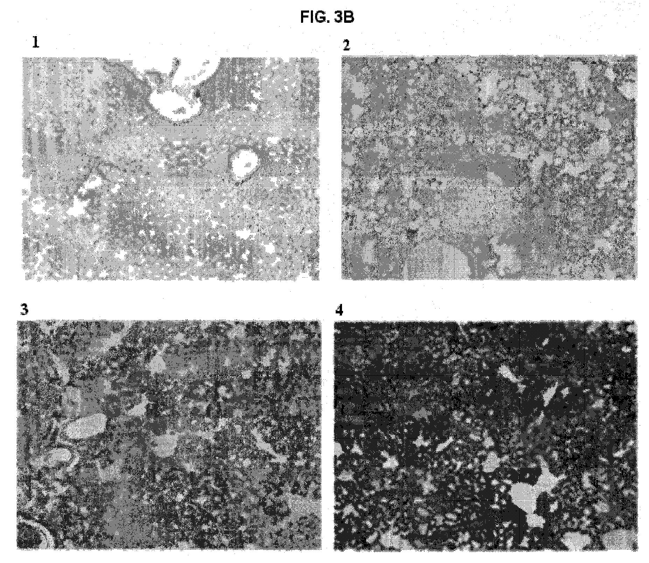

Figure 3 is (A) a typical representation of H&E sections of skin and underlying tissue from untreated control (slide 1), vehicle treated (slides 2 & 3: 1hr and 72 hr post dosing, respectively), and h-AT treated (slides 4 & 5: α1-AT 1hr and 72 hr post dosing, respectively) rabbits; and (B) a typical representation of immunohistology sections of lungs stained with primary monoclonal antibody to h-AT followed by a secondary monoclonal antibody to hAT-antibody complex and coupled to a diaminobenzedine chromophore and counter stained with hematoxyline (slides 1 & 2: Vehicle 1hr post dosing; slides 3 & 4: α1-AT 1hr and 72hr post dosing, respectively).
Figure 4 is a graph showing plasma human alpha-1 antitrypsin after IV and SC (± hyaluronidase) administration in rabbits.

### DETAILED DESCRIPTION

In accordance with the present invention, it has been surprisingly discovered that subcutaneous administration of α1-AT is an inherently simple and novel method of providing α1-AT to a subject (e.g., a human or an animal). A dosing regimen comprising subcutaneous administration of α1-AT is surprisingly well-suited for providing to the subject a therapeutically or prophylactically effective amount of α1-AT. Subcutaneous administration (e.g., subcutaneous injection using a needle) does not require skilled health care providers to administer the drug. A dosage unit may optionally be administered from a prepackaged device that punctures the skin to the correct extent and releases the dosage. Moreover, subcutaneous administration is the cause of less discomfort to the subject than is normal with intramuscular and intravenous injections as well as with rectal suppositories. In summary, subcutaneous administration of α1-AT as described herein provides an improvement for treating or preventing disorders or diseases associated with α1-AT deficiency.

The term " α1-AT ," as used herein, is intended to be broad unless specifically stated otherwise. The term refers to all naturally occurring polymorphs of α1-AT. The term also includes functional fragments of α1-AT, chimeric proteins comprising α1-AT or functional fragments thereof, homologs obtained by analogous substitution of one or more amino acids of α1-AT, and species homologs. The term also refers to all α1-AT polypeptides that are a product of recombinant DNA technology including an α1-AT that is a product of transgenic technology. For example, the gene coding for α1-AT can be inserted into a mammalian gene encoding a milk whey protein in such a way that the DNA sequence is expressed in the mammary gland as described in U.S. Patent No. 5,322,775. The term also refers to all α1-AT proteins synthesized chemically by means well known in the art such as, e.g., solid-phase peptide synthesis. The term also refers to α1-AT prepared from plasma. The term also refers to α1-AT that is commercially available. For example, Prolastin® (Talecris Therapeutics, Inc., Research Triangle Park, NC), Aralast™ (Baxter International, Inc., Deerfield, IL) and Zemaira® (CSL Behring, Kankakee, IL) are prepared from pools of human plasma. α1-AT from human plasma also is commercially available from Sigma-Aldrich Chemical Co. as products A9024, A6150, and 10814 (Sigma-Aldrich Chemical Co., Milwaukee, WI). α1-AT also is available from PPL Therapeutics, East Mains, Ormiston, East Lovian, EH35 5NG, Scotland. U.S. Patent No. 6,974,792 describes a method of preparing α1-AT.

The term "subcutaneous," as used herein, refers to below the skin (e.g., in the connective tissue underlying the dermis and above the facia of the muscle tissue).

A "therapeutically effective amount" refers to an amount effective, at dosages and for periods of time necessary, to achieve the desired therapeutic result, such as, for example, reduction or inhibition of emphysema associated with congenital deficiency of α1-AT. A therapeutically effective amount of α1-AT may vary according to factors such as the disease state, age, sex, and weight of the individual subject, and the ability of the α1-AT to elicit a desired response in the subject. Dosage regimens may be adjusted to provide the optimum therapeutic response. A therapeutically effective amount is also one in which any toxic or detrimental effects of α1**-**AT are outweighed by the therapeutically beneficial effects.

A "prophylactically effective amount" refers to an amount effective, at dosages and for periods of time necessary, to achieve the desired prophylactic result, such as, for example, preventing or inhibiting emphysema associated with congenital deficiency of α1-AT. A prophylactically effective amount can be determined as described above for the therapeutically effective amount.

The dose and frequency of subcutaneous administration can be readily tailored by one of ordinary skill in the art to provide a dosing regimen that is therapeutically or prophylactically effective.

Subcutaneous administration, therefore, refers to the delivery of a desired dosage of α1-AT below the skin via a medication delivery device. The medication delivery device can penetrate the epidermis of the individual to be treated, and results in introducing the desired dosage of α1-AT into the tissues of the individual. The delivery device of the present invention may include, but is not limited to any traditional hypodermic needle injectors, air-powered needle-less injectors, jet injectors, or gas-pressured needle-less injectors (see, *e*.*g*., U.S. Pat. Nos. 5,730,723, 5,891,086, 5,957,886, and 5,851,198).

In one embodiment, the subject is a human, wherein the site of subcutaneous administration includes, but is not limited to supra scapular, upper chest, upper thigh, and combinations thereof.

Examples of disorders and diseases associated with or caused by lower than normal plasma α1-AT levels and for which the present embodiments directed to subcutaneous delivery can be especially useful include, without limitation, lung disease such as chronic obstructive pulmonary disease (COPD) (*e*.*g*., emphysema), liver disease, vascular disease (e.g., intracranial aneurysms, arterial fibromuscular dysplasia, severe bleeding disorders, and hypertension), panniculitis, eye disease (e.g., anterior uveitis), systemic necrotizing vasculitis, and Wegener's granulomatosis.

One factor that may be considered when determining a therapeutically or prophylactically effective amount of α1-AT for subcutaneous administration is the concentration of functionally active α1-AT expressed in a biological compartment of the subject, such as, for example, in the lower respiratory tract or the epithelial lining fluid (ELF) of the subject. Another factor that also may be considered when determining a therapeutically or prophylactically effective amount of α1-AT for subcutaneous administration is the pharmacology (*e*.*g*., pharmacokinetics) of α1-AT.

The pharmacokinetics of α1-AT refers to the concentration or levels of α1-AT in the subject's blood. Pharmacokinetic parameters or measures of α1-AT levels in the blood include the area under the curve (AUC), Cₘᵢₙ (*i*.*e*., trough), and Cₘₐₓ. The AUC is the total exposure of α1-AT in the subject's blood over a fixed dosing period (*e*.*g*., 8, 12, and 24 hours). The Cₘᵢₙ *(i.e.,* trough level) is the lowest blood level of α1-AT during a fixed dosing period. The Cₘₐₓ is the highest or peak level achieved by α1-AT in the subject's blood over a fixed dosing period.

It is to be noted that dosage values may vary with the severity of the condition to be alleviated. It is to be further understood that for any particular subject, specific dosage regimens can be adjusted over time according to the individual need and the professional judgment of the caregiver, and that dosage ranges set forth herein are exemplary only and are not intended to limit the scope or practice of the invention.

Subcutaneous dosage regimens may be adjusted to provide the optimum therapeutic or prophylactic response. For example, a single subcutaneous bolus may be administered, several divided doses may be administered subcutaneously over time, or the dose may be proportionally reduced or increased as indicated by the requirement of the therapeutic or prophylactic situation. Further, one or more subcutaneous doses can be administered as part of a regimen comprising other modes of α1-AT administration including, but not limited to, intravenous administration.

In one aspect, α1-AT is for use in a method for treating or preventing a disorder or disease associated with α1-AT deficiency in a subject.

α1-AT is to be provided subcutaneously in a therapeutically or prophylactically effective amount.

For example, the therapeutically or prophylactically effective amount that is to be administered subcutaneously can be sufficient to achieve or maintain an α1-AT blood (*e*.*g*., plasma) trough level above a target threshold level. In one embodiment, the target threshold level is at least about 80, 85, 90, 100, 110, 120, 130, 140, 150, 160, 170, 180, 190, and 200 mg/dL.

In another embodiment, wherein the subject is human, the therapeutically or prophylactically effective amount is sufficient to achieve or maintain an α1-AT blood trough level of at least about 50 mg/dL. In other embodiments, the therapeutically or prophylactically effective amount of α1-AT is sufficient to maintain a blood α1-AT trough level of at least about 80 mg/dL. In one embodiment, the step of providing comprises a plurality of subcutaneous injections of a composition comprising a concentration of α1-AT.

The dose and frequency of subcutaneous administration of α1-AT may be tailored to the individual subject. Accordingly, both the dose and frequency can vary.

In one embodiment, the dose to be subcutaneously administered is at least about 70 mg to about 300 mg, about 80 mg to about 250 mg, about 90 mg to about 200 mg, and about 100 mg to about 150 mg per kg of body weight of subject per administration. The dose is about 60 mg to about 300 mg per kg of body weight of the subject.

Doses may be subcutaneously administered at such frequencies and for as long as is deemed necessary and safe, as is readily ascertained by standard tests by the caregiver (*e*.*g*., physician or veterinarian) depending upon the specific disorder or disease being treated.

In one embodiment, the dose is subcutaneously administered at least once per a time unit, illustratively, at least once, twice, three times, four times, five times, six times, 7 times, 8 times, 9 times, 10 times, 15 times, 20 times, 30 times, 40 times, 50 times, and so on per a time unit. In another embodiment, the time unit is an hour, a day, a week, every two weeks, every three weeks, a month, every 1, 2, 3, 4, 5, and 6 months, etc.

Where appropriate, the dosages and frequencies of subcutaneous administration may be adjusted upward or downward. In other embodiments, subcutaneous administration may be performed before, simultaneous with, or after other modes of α1-AT administration such as, for example, intramuscular or intravenous administration.

In one embodiment, the present invention provides a method for treating or preventing a disorder or disease associated with α1-AT deficiency in a subject in need of such treatment or prevention. The method comprises providing, subcutaneously, a therapeutically or prophylactically effective amount of α1-AT to the subject. In one embodiment, the therapeutically or prophylactically effective amount of α1-AT is at least about 72 mg/kg of body weight per week. In another embodiment, the step of providing comprises one or more subcutaneous injections of the subject, wherein the one or more injections each comprise a volume of a composition comprising a concentration of α1-AT, wherein each of the one or more injections is subcutaneously administered over a duration of administration. In some embodiments, the therapeutically or prophylactically effective amount of α1-AT is provided via a plurality of subcutaneous injections that are repeated week. The plurality of subcutaneous injections can be administered on the same day or on at least two different days of each week.

A variety of different α1-AT containing formulations can be used in accordance with the present invention. The active ingredient within such formulations is α1-AT, which can be human or non-human recombinant α1-AT but, as indicated above, also may include α1-AT extracted from plasma sources. Although the α1-AT is generally present by itself as the sole active ingredient, the α1-AT may be present with one or more additional active ingredient.

Regardless of the active ingredient, there can be different types of α1-AT formulations which can be used in connection with the present invention. All of the formulations include α1-AT and, optionally, a pharmaceutically acceptable carrier suitable for subcutaneous administration. In one embodiment, the formulation is a phosphate buffered saline (PBS) composition comprising a concentration of α1-AT. In another embodiment, the concentration is at least about 50 mg/ml.

Thus, any formulation which makes it possible to subcutaneously administer α1-AT to a subject can be used in connection with the present invention including formulations that also can be administered intravenously. Specific information regarding formulations which can be used in connection with subcutaneous delivery are described within Remington's Pharmaceutical Sciences, A. R. Gennaro editor (latest edition) Mack Publishing Company. Regarding insulin formulations, it is also useful to note Sciarra et al. [Journal of Pharmaceutical Sciences, Vol. 65, No. 4, 1976].

The α1-AT can be lyophilized in the container in which reconstitution of the α1-AT protein is to be carried out in order to avoid a transfer step. Generally, lyophilization will result in a lyophilized formulation in which the moisture content thereof is less than about 5%, and preferably less than about 3%. And, typically, when it is time to subcutaneously administer the α1-AT to the subject, the lyophilized formulation may be reconstituted with a suitable diluent to achieve the desired α1-AT concentration.

Reconstitution generally takes place at a temperature of about 25°C to ensure complete hydration, although other temperatures may be employed as desired. The time required for reconstitution will depend, e.g., on the type of diluent, amount of excipient(s) and protein. Exemplary diluents include, but are not limited to, sterile water, bacteriostatic water for injection (BWFI), a pH buffered solution (*e*.*g*. phosphate-buffered saline), sterile saline solution, and Ringer's solution or dextrose solution. The diluent optionally contains a preservative. The amount of preservative employed is determined by assessing different preservative concentrations for compatibility with the protein and preservative efficacy testing.

The reconstituted formulation is to be administered to the subject, such as, for example, a human, in accordance with known methods, by subcutaneous (*i.e.* beneath the skin) administration. For such purposes, the formulation may be injected using a syringe. However, other devices for administration of the formulation are available such as injection devices (*e*.*g*. the Inject-case™ and Genject™ devices); injector pens (such as the GenPen™); needleless devices (*e*.*g*. MediJector™ and BioJector™); and subcutaneous patch delivered systems. The protein may be administered as the sole treatment or in conjunction with other drugs or therapies. Subcutaneous placement of a winged infusion set, for example, can be used to minimize repeated needle injections.

In one embodiment, the concentration of α1-AT in the formulation for subcutaneous administration is at least about 0.1 mg/ml, illustratively, at least about 0.1, 1, 10, 100, 1000 mg/ml. Moreover, the specific activity of α1-AT in the formulation for subcutaneous administration can vary. In one embodiment, the specific activity is at least about 0.05 mg active α1-AT per mg total protein, illustratively, at least about 0.05, 0.1, 0.2 mg, 0.3mg, 0.4 mg, 0.5 mg, 0.6 mg, 0.7 mg, or more active α1-AT per mg total protein.

Generally, an individual subcutaneous administration of α1-AT comprises a volume of an α1-AT formulation, wherein the volume is administered over a duration of administration. The volume will of course depend in part on the concentration of α1-AT in the formulation to be administered. Moreover, the duration of administration can depend on one or more factors including, but not limited to the volume that is administered and the subject's discomfort level. For example, a dose having a relatively large volume can be provided as separate smaller volumes that can be administered at the same or a different time to the same or a different subcutaneous site (*e*.*g*., simultaneous administration of two smaller volumes to two different subcutaneous sites).

In one embodiment, the volume that is administered at a subcutaneous site is less than about 3 mL. Preferably, the duration of administration of the volume is at least about 0.5 second (sec), illustratively, at least about 0.5 sec, 1 sec, 10 sec, 20 sec, 30 sec, 40 sec, 50 sec, 1 minute (min), 2 min, 3 min, 4 min, 5 min, 10 min, 20 min, 30 min, 40 min, 50 min, and 1 hr.

In other aspects, α1-AT can be administered subcutaneously for a variety of applications including human and veterinary therapies, either alone or in combination with one or more reagents, e.g., a reagent capable of enhancing the pharmacokinetics and/or pharmacodynamics of the subcutaneously administered α1-AT. For example, in one embodiment, the one or more reagents are to be administered to a subject simultaneously with al-AT, for example by way of a single sterile pharmaceutical composition comprising α1-AT and the one or more reagents. In other embodiments, the one or more reagents are administered at a time prior to or following α1-AT administration.

The one or more reagents can be administered by injection, e.g., parenteral injection including, but not limited to, subcutaneous, intramuscular, intraorbital, intracapsular, and intravenous injection. The route of administration and the amount of the one or more reagents administered can vary according to various subject variables including size, weight, age, disease severity, and responsiveness to therapy. Methods for determining the appropriate route of administration and dosage of the one or more reagents can generally be determined on a case-by-case basis. Such determinations are routine to one of ordinary skill in the art (see, for example; Harrison's Principles of Internal Medicine, 11th Ed., 1987).

In one embodiment, the one or more reagents is a hydrolase such as, for example, a glycosidase. Illustrative non-limiting example of a glycosidases includes hyaluronidase, α-amylase, β-amylase, keratanase, dextranase, endoglycoceramidase II, chitosanase, chitinase, thioglucosidase, pectinase, pectolyase, lysozyme , neuraminidase, α-glucosidase, β-glucosidase, α-galactosidase, β-galactosidase, α-mannosidase, invertase, trehalase, amyloglucosidase, β-glucuronidase, hyaluronidase, cellulase, hesperidinase, pullulanase, α-N-acetylgalactosaminidase, α-L-fucosidase, β-N-acetylglucosaminidase, β-N-acetylhexosaminidase, β-glucanase, laminarinase, isoamylase, inulinase, xylanase, agarase, endoglycosidase H, endoglycosidase F2, endoglycosidase F3, endoglycosidase H, endoglycosidase F1, O-glycosidase, Fpg protein, NADase, diastase, and xylanase.

In some embodiments, the glycosidase is a hyaluronidase. For example, the hyaluronidase can be an enzyme that is capable of cleaving β-N-acetylhexosamine-(1-4)-glycosidic bonds in hyaluronic acid, chondroitin, and/or chondroitin sulfates. For example, the hyaluronidase can be administered as an adjuvant to increase the absorption of α1-AT thereby enhancing its pharmacokinetics and/or pharmacodynamics. Without being held to any particular theory, it is believed that hyaluronidase can act as a spreading or diffusing substance which modifies the permeability of connective tissue through the hydrolysis of hyaluronic acid, a polysaccharide found in the intercellular ground substance of connective tissue, and of certain specialized tissues, such as the umbilical cord and vitreous humor. Hyaluronidase at least can hydrolyze hyaluronic acid by splitting the glucosaminidic bond between C1 of the glucosamine moiety and C4 of glucuronic acid. It is believed that this can temporarily decrease the viscosity of the cellular cement and promote diffusion of injected fluids or of localized transudates or exudates, thus facilitating their absorption. The rate of diffusion can be proportionate to the amount of enzyme, and the extent can be proportionate to the volume of solution. Knowledge of the mechanisms involved in the disappearance of injected hyaluronidase is limited, however, it is believed that the blood of a number of mammalian species brings about the inactivation of hyaluronidase.

The specific dosage appropriate for administration can be readily determined by one of ordinary skill in the art and/or according to the factors discussed herein. In addition, one of ordinary skill in art will recognize that the estimates for appropriate dosages in humans can be extrapolated from determinations of the level of enzymatic activity of the enzyme *in vitro* and/or dosages effective in animal studies. For example, wherein X units (U) of hyaluronidase can be effective in enhancing the pharmacokinetics and/or pharmacodynamics of a subcutaneously administered α1-AT regimen in a rabbit weighing about 3 kg and, given this information, the corresponding hyaluronidase dosages in a human weighing about 75 kg could be about 25 times (*e.g*., about 25 times X units) the effective dose in the rabbits.

A number of hyaluronidase injections are known such as, for example, formulations marketed as Amphadase™ (150 units/mL injectable solution), Hydase™ (150 units/mL injectable solution), Hylenex™ (150 units/mL injectable solution), Vitrase™ (200 units/mL injectable solution), and Vitrase™ (6200 units injection). For example, according to one manufacturer, Amphadase™ (Amphastar Pharmaceuticals, Inc., Rancho Cucamonga, CA) is a preparation of purified bovine testicular hyaluronidase supplied as a sterile, colorless, ready for use solution, and absorption and dispersion of other injected drugs may be enhanced by adding 50-300 Units Amphadase™, typically 150 U Amphadase™, to the injection solution. Each vial of Amphadase™ contains 150 USP units of hyaluronidase per mL with 8.5 mg sodium chloride, 1 mg edetate disodium, 0.4 mg calcium chloride, monobasic sodium phosphate buffer, and not more than 0.1 mg thimerosal (mercury derivative). The ready for use solution is clear and colorless with an approximate pH of 6.8 and an osmolality of 295 to 355 mOsm.

α1-AT may be subcutaneously administered to a subject in a composition comprising al-AT in combination with an effective amount of a hyaluronidase, wherein the effective amount is sufficient to increase absorption, dispersion, or both of α1-AT. In one embodiment, the hyaluronidase is administered contemporaneously with α1-AT. In another embodiment, the hyaluronidase is administered contemporaneously with α1-AT, wherein the composition further comprises the hyaluronidase. In some embodiments, the hyaluronidase is administered contemporaneously with α1-AT, wherein the composition comprising α1-AT is administered at a site different from the site of administration of the hyaluronidase.

In other embodiments, the present specification illustrates a pharmaceutical composition comprising α1-AT, a hyaluronidase, and a pharmaceutically acceptable carrier, wherein the composition is suitable for subcutaneous administration to a subject. In one embodiment, the subject is a human. The pharmaceutical composition can be in the form of a lyophilized formulation.

An article of manufacture may be provided which contains the compositions or lyophilized formulations of the present invention and provides instructions for their reconstitution and/or use. The article of manufacture comprises a container. Suitable containers include, for example, bottles, vials (*e*.*g*. dual chamber vials), syringes (such as dual chamber syringes) and test tubes. The container may be formed from a variety of materials such as glass or plastic. The container holds the compositions or lyophilized formulations and the label on, or associated with, the container may indicate directions for reconstitution and/or use. For example, the label may indicate that the lyophilized formulation is reconstituted to protein concentrations as described above. The label may further indicate that the formulation is useful or intended for subcutaneous administration. The container holding the formulation may be a multi-use vial, which allows for repeat administrations (*e*.*g*. from 2-6 administrations) of the reconstituted formulation. The article of manufacture may further comprise a second container comprising a suitable diluent (*e*.*g*. BWFI). The article of manufacture may further include other materials desirable from a commercial end user standpoint, including other buffers, diluents, filters, needles, syringes, and package inserts with instructions for use.

### EXAMPLES

### Example 1 Plasma Bioavailability of Subcutaneously Administered Human α1-AT

To determine the plasma bioavailability of α1-AT following subcutaneous (SC) administration (and, to compare it with intravenous injection of α1-AT), single administration of two subcutaneous dose levels were examined and compared to a single intravenous administration of α1-AT.

New Zealand White rabbits (∼3 kg body weight) were acclimated for at least 1 week prior to use. Food and water were provided ad libitum. Rabbits were monitored daily for well being.

Rabbits were assigned into Groups A, B or C (N = 5 each) of similar body weight. Rabbits were treated with acepromazine (1.0 mg/kg body weight) by SC injection to facilitate vasodilation in the ears. Nair® or other depilatory was applied to the ear to facilitate removal of hair and to expose the blood vessels. Five to ten minutes later, the Nair® and hair were removed by wiping with wet gauze sponges until all cream was removed. Ears were dried and the local anesthetic, lidocaine (1%) cream was applied. After 15 minutes, the ear was cleaned with an alcohol swab and for the intravenous group (IV group), α1-AT (50 mg/ml) was administered (200 mg/kg body weight) via the ear vein.

For the subcutaneous groups (SC-1 and SC-2 groups), the hair at two sections (2" x 2") on the dorsal side was shaved. α1-AT (200 mg/kg body weight (SC-1 group); 240 mg/kg body weight (SC-2 group)) was administered subcutaneously at the two sites using a 25-G monoject needle attached to a syringe. Before infusion, the piston of the syringe was pulled back to ensure that the tip of the needle was not in a blood vessel. After the infusion, visual observation was made to ensure lack of leakiness.

Blood was collected at zero time (pre-dose), 5min, 3hr, 6hr, 9hr, 1, 2, 3, 4, 6, 8, and 10 days. A blood sample of 1.5 ml from the arterial catheter was collected using a syringe charged with 150ul of 3.8% sodium citrate and transferred to a micro centrifuge tube.

After the initial blood collection, animals were returned to their holding cages. At each subsequent collection time the animals were returned to their restraining cages, blood sample was taken as described above, and animals were returned to their holding cages. Before subsequent blood draws rabbits were sedated with 1 mg/ml acepromazine and 1.5ml blood sample collected directly from the ear artery using a 22g needle and 3ml syringe charged with 3.8% sodium citrate. Rabbits were euthanized at the end of the study by intravenous infusion of pentobarbital. All rabbits were euthanized in accordance with Public Health Service policies on the humane care of laboratory animals. Any adverse effects on the rabbits were addressed in strict accordance with approved veterinary protocols at North Carolina State University, College of Veterinary Medicine.

Blood samples were centrifuged after collection at 3000 RPM in a tabletop centrifuge, plasma samples were frozen in dry ice and stored at -80° C. The plasma samples were analyzed for h-AT by immunonephelometry.

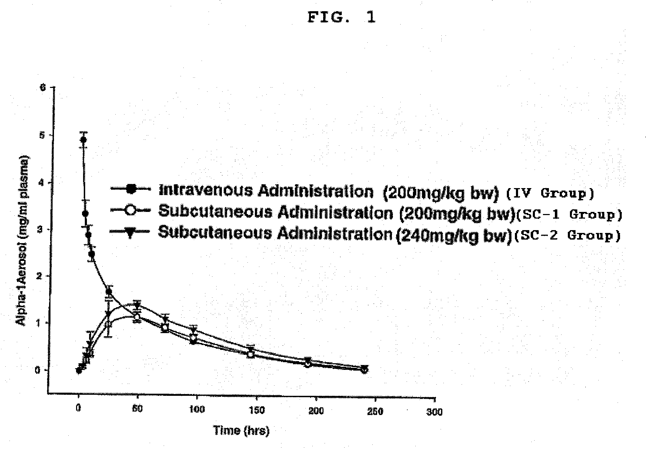

As shown in Figure 1, the plasma elimination profile of intravenously administered α1-AT *(i.e.,* IV group) follows a biphasic pattern with a rapid initial alpha phase and a slow beta phase with a terminal elimination half-life (τ_{1/2}) of 53 hours. The subcutaneously administered α1-AT *(i.e.,* SC-1 and SC-2) reached Cₘₐₓ in about 48hrs. The fractional availability (F) derived from the area under concentration curve's (AUC's) of SC-1 (*i*.*e*., 100% IV dose) and SC-2 *(i.e.,* 120% of IV dose) group were, respectively, 27% lower (P<0.05) and 8% lower (not statistically significant) compared to the IV group. The results are summarized in Table 1.

**Table 1: Plasma Profile**

| **Group** | **AUC (hr·mg/ml) Mean ± SD** | **F Mean ± SD** |
|---|---|---|
| IV | 179.0 ± 11.2 | 1.00 ± 0.06 |
| SC-1 | 131.4 ± 13.1* | 0.73 ± 0.07* |
| SC-2 | 164.6 ± 21.0 | 0.92 ± 0.12 |

| | | |
|---|---|---|
| *Statistically significant from the IV group at P<0.05 | | |

### Example 2

### Plasma Bioavailability After Repeated Subcutaneous Administration of Human α1-AT

Plasma bioavailability of α1-AT following three repeated subcutaneous (SC) administrations were examined.

Rabbits were prepared as above and assigned to three groups and dosed on day 0, 2, 4, and 6. Group 1 rabbits *(i.e.,* SC-3) were dosed at 50mg/kg, Group 2 rabbits *(i.e.,* SC-4) were dosed at 60mg/kg, and Group 3 rabbits *(i.e.,* SC-5) were dosed at 70mg/kg. Blood samples were collected at1, 2, 3, 4, 5, 6, 7, 8, 9, 10, and 12 days post dosing and processed as above for analysis.

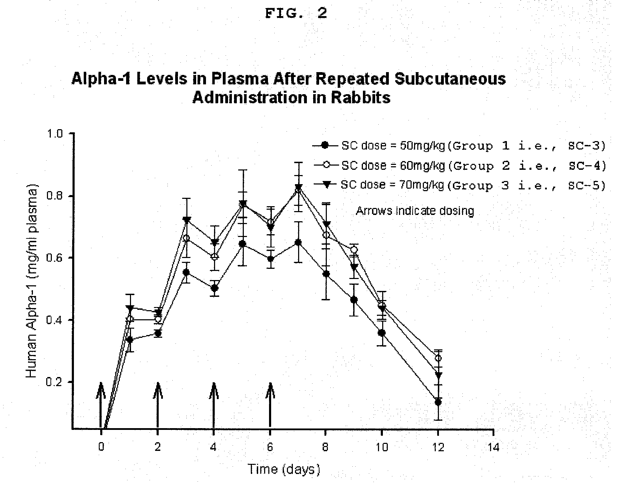

As shown in Figure 2, the plasma levels of subcutaneously administered α1-AT in all the three groups *(i.e.,* SC-3, SC-4, and SC-5) continued to rise with repeated administration up to day 7 and thereafter the levels gradually declined towards baseline by day 12. The AUC of SC-3, SC-4, and SC-5 were 127 ± 11, 159 ± 8, and 162 ± 12, respectively. The fractional availability (F), which was determined by comparing the AUC to the single dose IV group from the experiment described in Example 1 above, was 0.71 ± 0.03, 0.89 ± 0.04, 0.91 ±0.07 for SC-3, SC-4, and SC-5, respectively. The F value for SC-3 was lower than SC-4 and SC-5 by about 20% (P<0.05). The results are summarized in Table 2.

**Table 2. Area Under the Curve and Fractional Availability (F)**

| **Group** | **AUC (hr·mg/ml) Mean ± SD** | **F Mean ± SD** |
|---|---|---|
| 1: SC-3 | 127 ± 11* | 0.71 ± 0.03* |
| 2: SC-4 | 159 ± 8 | 0.89 ± 0.04 |
| 3: SC-5 | 162 ± 12 | 0.91 ± 0.07 |

| | | |
|---|---|---|
| *P<0.05 compared to smgle dose IV at 200 mg/kg described in Example 1. | | |

### Example 3

### Injection Site Histopathology of SC Administered Human α1-AT

To determine injection site histopathology and the presence of human α1-AT in lung tissue, immunohistochemistry was performed after repeated subcutaneous administration of human alpha-1 antitrypsin (h-AT) in rabbits.

All animal procedures were approved by Institutional Animal Care and Use Committee (IACUC) at North Carolina State University. Male New Zealand White rabbits, after arrival at the animal facility, were acclimated for at least 1 week prior to use. During this acclimation period, each animal was placed in a restrainer, three times / week for a minimum of 15 minutes to acclimate the animal to the restrainer. Food and water were provided ad libitum. Rabbits were monitored daily for well being.

Animals were weighed and assigned to groups (n = 3) of approximately equal body weight as shown in Table 3.

**Table 3: Study design**

| **Groups** | **N** | **Treatments** | **Total Volume (mL)** | **Tissue Collection Time** |
|---|---|---|---|---|
| Group 1-A | 3 | Alpha-1 Antitrypsin Daily SC administration for 3-days | 10ml | 1hr after last dose |
| Group 1-B | 3 | Alpha-1 Antitrypsin Daily SC administration for 3-days | 10ml | 3-day after the last dose |
| Group 2-A | 3 | Vehicle (PBS) Daily SC administration for 3-days | 10ml | 1hr after last dose |
| Group 2-B | 3 | Vehicle (PBS) Daily SC administration for 3-days | 10ml | 3-day after the last dose |
| Group 3-A | 1 | No Treatment | 10ml | At same time as group A |

The day prior to the start of the study, an area on the left flank of the animals was clipped from the dorsal midline extending 4-cm ventral and from the tip of the last rib to the wing of the ileum. On the day of the study, animals were sedated with SC injection of Acepromazine (1mg/kg) and 15-minutes later, test articles (10ml h-AT - 56mg/ml - Lot # T06AU02) or vehicle (phosphate buffer saline) were administered by SC injection by a 23-gauge butterfly catheter. The animal skin was immobilized by gentle traction and the butterfly needle from the catheter was inserted into the space between the skin and the underlying subcutaneous tissue to ensure that there is minimal movement of the needle to avoid injury of the tissues.

The test articles were administered SC at the same site for each rabbit and the injection site and tissue distortion borders were marked with a marker. The injections as outlined above were repeated each day for three consecutive days.

Tissue collection times are outlined in the Table 3 above. The animals were anesthetized with ketamine (50 mg/kg b/w) and xylazine (10mg/kg b/w) intramuscularly (IM) and euthanized by pentobarbital (120 mg/kg or 7mls per 3 kg rabbit) administered intravenously (IV).

For lung tissue analysis, the thoracic cavity was opened and the lungs removed. The trachea was catheterized and lungs were inflated with 50-100ml buffered Formaldehyde, pH 7.4. After inflation lungs were placed in formaldehyde and processed for immunohistochemisty within 72 hours. h-AT specific staining was performed with a primary monoclonal antibody followed by a secondary monoclonal antibody to h-AT-antibody complex and coupled to a diaminobenzedine chromophore and counter stained with hematoxyline.

For skin tissue analysis, the entire left flank was harvested and a 3"x 3" section of skin and underlying muscle wall was incised, pinned to Styrofoam and placed in formaldehyde. The tissue was marked dorsal, ventral, cranial and caudal with a marker. The dorso-cranial corner of the squared tissue section was cut off to further provide orientation for sectioning and histopathology.

The results of injection site histology of skin and underlying tissues and lung immunohistochemistry are shown in Figure 3. Figure 3A, slides 1-5 shows that there was no indication of inflammation or signs of injury in rabbits treated with h-AT or vehicle compared to untreated control suggesting that repeated SC administration of h-AT did not alter skin and underlying tissue histology. As shown in Figure 3B, lungs from rabbits treated with vehicle (slides 1 & 2) did not show dark staining indicating the lack of h-AT, however, rabbits treated with h-AT (slides 3 & 4) showed dark staining indicating the presence of h-AT throughout the lung tissue including epithelial, endothelial, and interstitial space. These results suggest that SC administration of h-AT is readily available in the lung tissue, the target organ in the treatment of h-AT deficiency.

### Example 4

### Effect of Hyaluronidase on Pharmacokinetics of Subcutaneously Administered Human Alpha-1 Antitrypsin

To determine the effect of the enzyme hyaluronidase on pharmacokinetics of subcutaneously administered α1-AT, rabbits were administered α1-AT with or without hyaluronidase.

Rabbits were weighted and assigned into Group A, B or C (n = 5) and the average body weight of each group was matched. Rabbits were acclimated for at least 1 week prior to study. During this acclimation period, each animal were placed in a restrainer, three times per week for a minimum of 15 minutes to acclimate animal to sitting in the restrainer. Food and water were provided ad libitum and rabbits monitored daily for well being. Each rabbit was placed in a restrainer, and drugs were administered as follows:
1. h-AT - Intravenous: Rabbits were treated with acepromazine (1.0 mg/kg bw) by SC injection to cause vasodilation of the veins and arteries in the ears. h-AT (Lot # T06Au02-56mg/ml - Talecris Biotherapeutics) was administered [200mg/kg bw10.7] as a bolus in the ear vein via a butterfly needle attached to the syringe.
2. Subcutaneous Administration: Rabbits were treated with acepromazine (1.0 mg/kg bw) by SC injection to cause vasodilation of the veins and arteries in the ears. The hair on the dorsal side (2" for 2") was shaved and h-AT (Lot # T06Au02- 56mg/ml - Talecris Biotherapeutics) was administered [h-AT: 200mg/kg10.7; h-AT + hyaluronidase: 200mg/kg (10.7 ml/3 kg rabbit) of h-AT + 3,000 U of Hyaluronidase ( H-6254 - Sigma-Aldrich, St. Louis, MO)] subcutaneously via a butterfly needle attached to a syringe. Before infusion, the piston of the syringe were pulled back to observe for blood. After the infusion, visual observation was made for lack of leakiness.

Blood were collected at zero time (pre-dose), 5min, 4hr, and 1, 2, 3, 4, 6, 8, & 10 days. A blood samples of 1.5 ml were collected from the medial ear artery using a syringe charged with 150ul of 3.8 % Sodium citrate and transferred to a microfuge tubes. Blood samples were centrifuged at 3000 RPM in a tabletop centrifuge, plasma aliquots frozen in dry ice and stored at -80 °C.

Samples were analyzed for human-AT by immunonephelometry. Data were analyzed by Microsoft Excel (Microsoft, Seattle, WA) and pharmacokinetic parameters such as area-under-the-curve (AUC), fractional availability (F), half-life (T1/2), maximal plasma concentration (Cₘₐₓ), and maximum time for peak plasma concentration (Tₘₐₓ), etc were determined by using WinNonlin 5.2 (Pharsight Corp, Mountain view CA). Graphs were plotted using Sigma Plot 8.0 (Systat Software, Inc, San Jose, CA). Data was subjected to statistical analysis using JMP 7.0 (SAS, Cary, NC).

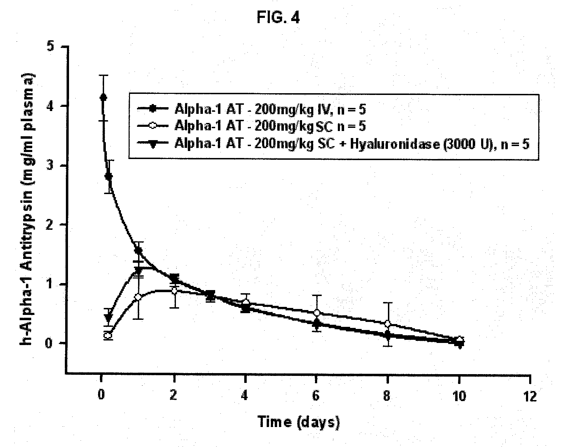

The levels of h-AT in rabbit plasma after IV and SC administration are shown in Figure 4. In the IV group, h-AT level was maximal at the first sampling point (5-minutes) and thereafter, levels declined rapidly with time up to day 10. The Tₘₐₓ (time to maximum levels) of h-AT in groups given h-AT SC alone or with hyaluronidase were 48hr and 24hr, respectively. These results show that hyaluronidase reduced the time to peak plasma h-AT concentration as compared to the group given h-AT alone. As shown in Table 4, the plasma AUC of h-AT administered by IV was 164.1 ± 14.3 hr*mg/ml (Mean ± SD) and that of groups given h-AT by SC with and without hyaluronidase were 131.3 ± 26.4 and 126.9 ± 7.7 hr*mg/ml, respectively.

**Table 4. Area Under the Curve (AUC)**

| **Group** | **AUC (hr·mg/ml) Mean ± SD** |
|---|---|
| Alpha-1 AT: IV | 164.1 ± 14.3 |
| Alpha-1 AT: SC | 131.3 ± 26.4* |
| Alpha-1 AT: SC (plus hyaluronidase) | 126.9 ± 7.7* |

| | |
|---|---|
| *P<0.05 compared to IV group. | |

Consistent with the results in Example 1 above, the results also show that equal doses of h-AT administered by IV and SC result in a lower AUC (20%) for the SC administration as compared to the IV AUC. Moreover, co-administration of hyaluronidase and h-AT resulted in 23% reduction of AUC as compared to the IV group and an almost similar reduction in AUC as that of the group administered SC h-AT alone. This suggests that hyaluronidase did not significantly affect the AUC as compared to the group administered SC h-AT alone. It can be concluded from the results of this study that hyaluronidase only affects the Tₘₐₓ but not AUC of h-AT administered SC in rabbits.

## Claims

1. Alpha-1 antitrypsin (α1-AT) for use in a method of treating or preventing a disorder or disease associated with α1-AT deficiency in a subject by subcutaneous administration, wherein the therapeutically or prophylactically effective amount of the subcutaneously administered α1-AT is at least about 120% of an intravenously administered therapeutically or prophylactically effective amount of α1-AT wherein the therapeutically or prophylactically effective amount of α1-AT is sufficient to maintain in the subject a blood α1-AT trough level of at least about 80 mg/dL, wherein the therapeutically or prophylactically effective amount of α1-AT is about 60 mg to about 300 mg of α1-AT per kg of body weight of the subject.

2. α1-AT for use according to claim 1, wherein the formulation is a lyophilized formulation to be reconstituted prior to administration.

3. α1-AT for use according to any one of claims 1 or 2, wherein the formulation further comprises a hydrolase.

4. α1-AT for use according to any one of claims 1 or 2, wherein the formulation further comprises a hyaluronidase.

## Patentansprüche

1. Alpha-1-Antitrypsin (α1-AT) zur Verwendung in einem Verfahren zum Behandeln oder Verhüten einer Störung oder Krankheit, die mit α1-AT-Mangel in einem Probanden verbunden ist, durch subkutane Verabreichung, wobei die therapeutisch oder prophylaktisch wirksame Menge des subkutan verabreichten α1-AT wenigstens ungefähr 120 % von einer intravenös verabreichten therapeutisch oder prophylaktisch wirksamen Menge von α1-AT beträgt, wobei die therapeutisch oder prophylaktisch wirksame Menge von α1-AT ausreicht, um in dem Probanden einen α1-AT-Talspiegel im Blut von wenigstens ungefähr 80 mg/dL aufrechtzuerhalten, wobei die therapeutisch oder prophylaktisch wirksame Menge von α1-AT ungefähr 60 mg bis ungefähr 300 mg α1-AT pro kg Körpergewicht des Probanden beträgt.

2. α1-AT zur Verwendung gemäß Anspruch 1, wobei die Formulierung eine lyophilisierte Formulierung ist, die vor der Verabreichung rekonstituiert werden soll.

3. α1-AT zur Verwendung gemäß einem der Ansprüche 1 oder 2, wobei die Formulierung außerdem eine Hydrolase umfasst.

4. α1-AT zur Verwendung gemäß einem der Ansprüche 1 oder 2, wobei die Formulierung außerdem eine Hyaluronidase umfasst.

## Revendications

1. Alpha-1 antitrypsine (α1-AT) pour utilisation dans une méthode de traitement ou de prévention d'un trouble ou d'une maladie associé à un manque de α1-AT dans un sujet par administration sous-cutanée, dans laquelle la quantité thérapeutiquement ou prophylactiquement efficace de α1-AT administrée en sous-cutané est d'au moins environ 120 % d'une quantité de α1-AT thérapeutiquement ou prophylactiquement efficace administrée en intraveineuse, dans laquelle la quantité thérapeutiquement ou prophylactiquement efficace de α1-AT est suffisante pour maintenir dans le sujet un niveau global de α1-AT dans le sang d'au moins environ 80 mg/dl, dans laquelle la quantité thérapeutiquement ou prophylactiquement efficace de α1-AT est d'environ 60 mg à environ 300 mg de α1-AT par kilogramme de poids corporel du sujet.

2. α1-AT pour utilisation selon la revendication 1, dans laquelle la formulation est une formulation lyophilisée devant être reconstituée avant l'administration.

3. α1-AT pour utilisation selon l'une quelconque des revendications 1 ou 2, dans laquelle la formulation comprend en outre une hydrolase.

4. α1-AT pour utilisation selon l'une quelconque des revendications 1 ou 2, dans laquelle la formulation comprend en outre une hyaluronidase.
